# EUROPEAN PATENT SPECIFICATION

(11) **EP 2 846 780 B1**
(45) Date of publication and mention of the grant of the patent: **18.07.2018**
(21) Application number: 13722217.0
(22) Date of filing: 30.04.2013
(51) Int. Cl.: A61K 9/48, A61K 31/4709, A61P 31/14

(54) **SOLUBILIZED CAPSULE FORMULATION OF 1,1-DIMETHYLETHYL [(1S)-1-{[(2S,4R)-4-(7-CHLORO-4METHOXYISOQUINOLIN-1-YLOXY)-2-({(1R,2S)-1-[(CYCLOPROPYLSULFONYL)CARBAMOYL]-2-ETHENYLCYCLOPROPYL}CARBAMOYL)PYRROLIDIN-1-YL]CARBONYL}-2,2-DIMETHYLPROPYL]CARBAMATE**
SOLUBILISIERTE KAPSELFORMULIERUNG VON 1,1-DIMETHYLETHYL-[(1S)-1-{[(2S,4R)-4-(7-CHLOR-4METHOXYISOCHINOLIN-1-YLOXY)-2-({(1R,2S)-1-[(CYCLOPROPYLSULFONYL)CARBAMOYL]-2-ETHENYLCYCLOPROPYL}CARBAMOYL)PYRROLIDIN-1-YL]CARBONYL}-2,2-DIMETHYLPROPYL]CARBAMAT
FORMULATION DE CAPSULE SOLUBILISÉE DE 1,1-DIMÉTHYLÉTHYL[(1S)-1-{[(2S,4R)-4-(7-CHLORO-4-MÉTHOXYISOQUINOLIN-1-YLOXY)-2-({(1R,2S)-1-[(CYCLOPROPYLSULFONYL)CARBAMOYL]-2-ÉTHÉNYLCYCLOPROPYL}CARBAMOYL)PYRROLIDIN-1-YL]CARBONYL}-2,2-DIMÉTHYLPROPYL]CARBAMATE

(30) Priority: 07.05.2012 US 201261643454 P
(43) Date of publication of application: 18.03.2015
(73) Proprietor: Bristol-Myers Squibb Holdings Ireland, Steinhausen (CH)
(72) Inventor: PERRONE, Robert Kevin, New Brunswick, New Jersey 08903 (US)
(74) Representative: Vögeli-Lange, Regina
(86) International application number: PCT/US2013/038770
(87) International publication number: WO 2013/169520

(56) References cited:
- EP-A1- 1 283 041
- Anonymus: "A Multiple Ascending Dose Study of BMS-650032 in HCV Infected Subjects", ClinicalTrials.gov , 11 June 2011 (2011-06-11), pages 1-3, XP002702909, Retrieved from the Internet: URL:http://clinicaltrials.gov/ct2/show/stu dy/NCT00722358 [retrieved on 2013-07-18] & Anonymous: "History of Changes and the ClinicalTrials.gov Archive site. NCT00722358", ClinicalTrials.gov , 18 July 2013 (2013-07-18), XP002703552, Retrieved from the Internet: URL:http://clinicaltrials.gov/ct2/archive/ NCT00722358 [retrieved on 2013-07-18]
- C. PASQUINELLI ET AL: "Single- and Multiple-Ascending-Dose Studies of the NS3 Protease Inhibitor Asunaprevir in Subjects with or without Chronic Hepatitis C", ANTIMICROBIAL AGENTS AND CHEMOTHERAPY, vol. 56, no. 4, 1 April 2012 (2012-04-01), pages 1838-1844, XP055071012, ISSN: 0066-4804, DOI: 10.1128/AAC.05854-11
- COLE ET AL: "Challenges and opportunities in the encapsulation of liquid and semi-solid formulations into capsules for oral administration", ADVANCED DRUG DELIVERY REVIEWS, ELSEVIER BV, AMSTERDAM, NL, vol. 60, no. 6, 21 December 2007 (2007-12-21), pages 747-756, XP022476862, ISSN: 0169-409X, DOI: 10.1016/J.ADDR.2007.09.009
- DATABASE BIOSIS [Online] BIOSCIENCES INFORMATION SERVICE, PHILADELPHIA, PA, US; 2012, ELEY T ET AL: "Improved Bioavailability and Mitigated Food Effect for Asunaprevir (ASV) Utilizing a Lipid-Based Formulation: Similar Exposure with 100mg BID Softgel Capsule (SGC) Relative to 200mg BID of Phase 2 Tablet", XP002703558, Database accession no. PREV201300130807

## Description

The present disclosure relates generally to a solubilized capsule dosage formulation for poorly water soluble pharmaceutical compounds that have shown low oral bioavailability with a significant food effect. In particular, the disclosure relates to a new solubilized capsule dosage formulation of asunaprevir, 1,1-dimethylethyl [(1*S*)-1-{[(2*S*,4*R*)-4-(7-chloro-4methoxyisoquinolin-1-yloxy)-2-({(1*R*,2*S*)-1-[(cyclopropylsulfonyl)carbamoyl]-2-ethenylcyclopropyl}carbamoyl)pyrrolidin-1-yl]carbonyl}-2,2-dimethylpropyl]carbamate, and to uses of the formulation for the treatment and/or inhibition of the hepatitis C virus and infections caused thereby.

The compound of formula (I), asunaprevir, 1,1-dimethylethyl [(1*S*)-1-{[(2*S*,4*R*)-4-(7-chloro-4methoxyisoquinolin-1-yloxy)-2-({(1*R*,2*S*)-1-[(cyclopropylsulfonyl)carbamoyl]-2-ethenylcyclopropyl}carbamoyl)pyrrolidin-1-yl]carbonyl}-2,2-dimethylpropyl]carbamate, is a selective NS3 protease inhibitor and is useful in the treatment of the hepatitis C virus (HCV). 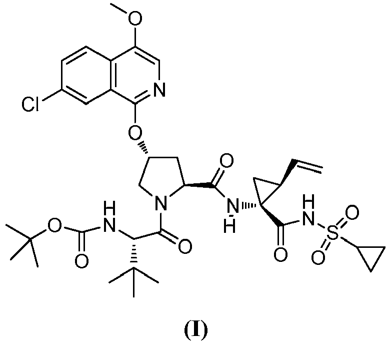 Asunaprevir and its preparation have been previously described in U.S. Patent No. 6,995,174, issued February 7, 2006, U.S. Patent No. 7,449,479, issued November 11, 1988, and U.S. Patent No. 7,915,291 which issued March 29, 2011.

Asunaprevir is a selective NS3 protease inhibitor of hepatitis C virus (HCV), and is intended for the oral treatment of hepatitis C infection. Numerous solid dosage forms of asunaprevir such as dry and wet granulations of amorphous or crystalline forms of the drug in a capsule or compressed into a tablet have shown low oral bioavailability with significant food effect. In addition, a significant reduction in the crystallinity of asunaprevir and formation of amorphous drug substance has been observed that results from various unit operations (e.g., roller compaction, tablet compression) in the manufacturing processes for capsules and tablets manufactured from dry and wet granulations and powders. Furthermore, the drug substance is susceptible to conversion from anhydrous to hydrate (or vice-versa) crystalline forms depending on the relative humidity of the environment during manufacture of the capsules or tablets from dry and wet granulations or powders. These factors significantly complicate the manufacturing process and control of the drug product due to potential variability in the drug form present in the formulation, requiring an increased level of monitoring, testing and controls.

The effects of single and multiple ascending doses of Asunaprevir in the form of capsules on HCV infected patients are described in Pasquinelli et al., Antimicrobial Agents and Chemotherapy, vol. 56, no.4, 1 April 2012, pages 1838-1844 and in "A Multiple Ascending Dose Study of BMS-650032 in HCV Infected Subjects", 11 June 2011 pages 1-3, retrieved from the Internet: URL http://clinicaltrials.gov/ct2/show/study/NCT00722358.

Suitably stable dosage forms of asunaprevir with significantly enhanced oral bioavailability and elimination of the food effect were obtained by solubilizing the drug in a mixture of excipients which include lipid components and filling into capsules. These dosage forms readily provide the drug in an emulsified-state at high concentrations which resist drug precipitation following dilution in aqueous milieu, significantly enhancing the oral absorption of asunaprevir in the fasted-state and providing comparable absorption in the fed-state.

The process for manufacturing the solubilized formulations of asunaprevir consists of significantly less unit operations compared to manufacturing processes for capsules or tablets from dry and wet granulations or powders. In addition, the solubilized-state of the drug eliminates the need for monitoring, testing and control of the drug substance for potential changes or variability in the form induced by the processes used for manufacturing capsules or tablets from dry and wet granulations or powders.

There is described hereinafter a formulation comprising a capsule comprising at least one active pharmaceutical ingredient comprising a solubilized compound having the formula (I)

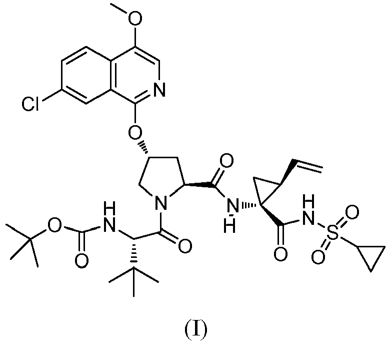

The formulation further comprises at least one solubilizer and optionally comprises at least one surfactant, and/or at least one stabilizer. In a second embodiment of the first aspect the at least one stabilizer is included in the range from about 0.01 to about 1% w/w.

The at least one active pharmaceutical ingredient is included in the range from about 0.1 to about 60% w/w, the at least one solubilizer is included in the range from about 1 to about 80% w/w, the at least one surfactant is included in the range from about 0 to about 60% w/w, and the at least one stabilizer is included in the range from about 0.01 to about 1% w/w.

The at least one active pharmaceutical ingredient is included in the range from about 1 to about 40% w/w, the at least one solubilizer is included in the range from about 10 to about 80% w/w, the at least one surfactant is included in the range from about 5 to about 40% w/w, and the at least one stabilizer is included in the range from about 0.02 to about 0.5% w/w.

The at least one active pharmaceutical ingredient is included in the range from about 1 to about 40% w/w, the at least one solubilizer is included in the range from about 5 to about 80% w/w, the at least one surfactant is included in the range from about 15 to about 40 % w/w, and the at least one stabilizer is included in the range from about 0.05 to about 0.2% w/w.

The at least one solubilizer may be comprised of medium-chain fatty acid triglycerides and a combination of medium-chain fatty acid mono- and diglycerides.

The at least one solubilizer may be polyoxyethylated glycerides.

The at least one surfactant may be polyoxyethylene sorbitan monooleate.

The at least one stabilizer may be butylated hydroxytoluene.

In a first aspect of the present invention, the present disclosure provides a formulation comprising:
(a) at least a compound of formula (I) 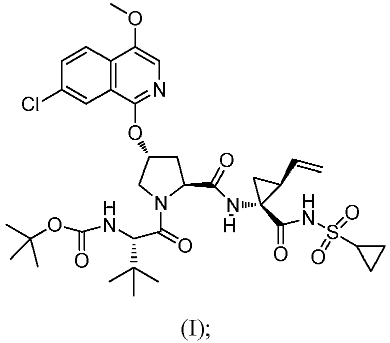
(b) at least one solubilizer which is medium-chain fatty acid triglycerides and a combination of medium-chain fatty acid mono- and diglycerides, wherein the at least one solubilizer is included in the range from 10 to 80% w/w;
(c) at least one surfactant which is polyoxyethylene sorbitan monooleate wherein the at least one surfactant is included in the range from 15 to 40% w/w; and
(d) at least one stabilizer which is butylated hydroxytoluene.

In a first embodiment of the first aspect the at least one active pharmaceutical ingredient is included in the range from 1 to 40% w/w.

In a second embodiment of the first aspect the at least one stabilizer is included in the range from 0.05 to 0.2% w/w.

There is described hereinafter a formulation comprising a capsule comprising at least one active pharmaceutical ingredient comprising a solubilized compound having the formula (I) 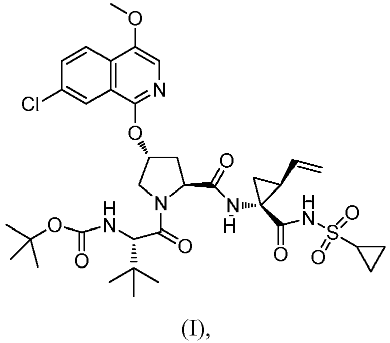 that is stable for at least six months at 40 °C and 75% relative humidity.

The at least one active pharmaceutical ingredient has at least about 98% potency retention.

The formulation may have less than 10%; less than 5%; less than 2%; less than 1% or less than 0.5% total degradants.

The formulation may comprise at least one stabilizer in an amount from about 0.01 to about 1.0% w/w.

There is described hereinafter a method of administering a formulation comprising orally administering to a fasted or fed mammalian subject a formulation comprising Compound (I) having the formula: 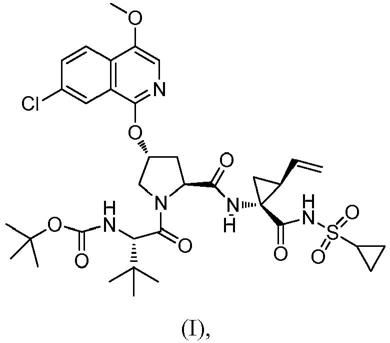 to provide a total blood plasma concentration profile of Compound (I), as measured by AUC at 24 hours after an initial dose of the composition, that is at least greater than about 50% of the total blood plasma concentration as measured by AUC at 24 hours of an initial dose of an orally administered solution comprising Compound (I).

The AUC may be at least greater than about 60%; greater than 70%; greater than 80%; greater than 90%; greater than 93% or greater than 95% of the AUC at 24 hours of the solution when orally administered regardless if the subject is fasted or fed.

There is described hereinafter a method of administering a formulation comprising orally administering to a fasted mammalian subject the composition comprising Compound (I) having the formula: 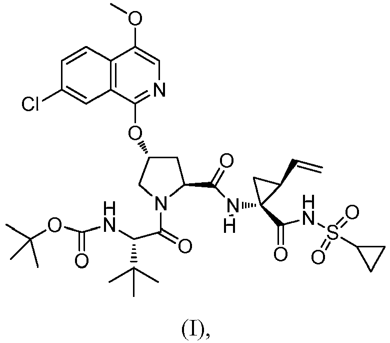 to provide a blood plasma concentration profile after an initial dose of the composition with a Cmax of Compound (I) after an initial dose of the composition that is at least greater than about 40%; greater than about 50% or greater than 60% of the Cmax of an orally administered solution comprising Compound (I).

The Tmax may be at least about 3 hours.

The formulation may be in a form of a capsule.

The formulation may be in a form of a solubilized capsule.

There is described hereinafter a method of administering an oral solid dosage composition comprising orally administering to a fasted mammalian subject the composition comprising at least one poorly soluble active pharmaceutical ingredient to provide a total blood plasma concentration profile as measured by AUC at 24 hours after an initial dose of the composition that is at least greater than about 50% of the total blood plasma concentration as measured by AUC at 24 hours of an initial dose of an orally administered solution comprising the at least one active pharmaceutical ingredient.

The active pharmaceutical ingredient exhibits a significant food effect.

The at least one active pharmaceutical ingredient may be included in the range from about 0.1 to about 60% w/w.

There is described hereinafter a method of administering a formulation comprising orally administering to a fasted mammalian subject the formulation comprising Compound (I) having the formula 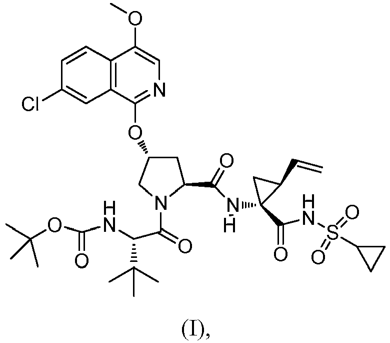 and has a fed to fasted ratio lower than at least about 2.

The fed to fasted ratio may be less than about 1.5; less than about 1.0 or less than about 0.75.

The formulation may be in a form of a capsule.

The formulation may be in a form of a solubilized capsule.

In a second aspect of the present invention, the present disclosure provides the formulation according to the first aspect for use in the treatment of an HCV infection

There is disclosed hereinafter a method of treating an HCV infection comprising the step of administering to a subject in need thereof a formulation comprising a capsule comprising at least one active pharmaceutical ingredient comprising a solubilized compound having the formula (I) 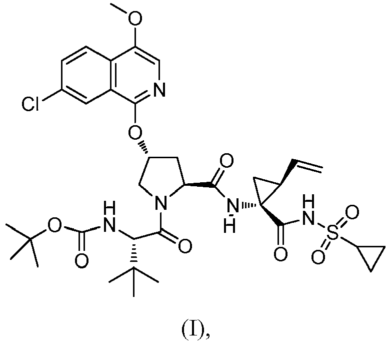 wherein the total dose of the compound of formula (I) administered to the subject is about 200 mg a day. In a first embodiment of the tenth aspect the compound of formula (I) is administered to the subject in doses of 100 mg two times a day. In a second embodiment of the tenth aspect the compound of formula (I) is administered to the subject in a dose of 100 mg one time a day.

There is disclosed hereinafter a method of treating an HCV infection comprising the step of administering to a subject in need thereof a formulation comprising a capsule comprising at least one active pharmaceutical ingredient comprising a solubilized compound having the formula (I) 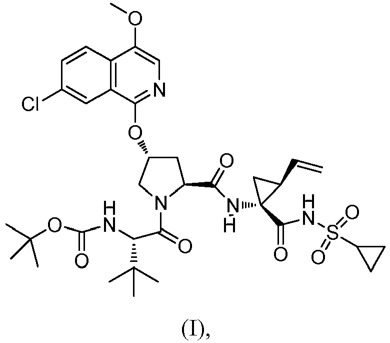 wherein the total dose of the compound of formula (I) administered to the subject is about 150 mg a day.

There is disclosed hereinafter a method of treating an HCV infection comprising the step of administering to a subject in need thereof a formulation comprising a capsule comprising at least one active pharmaceutical ingredient comprising a solubilized compound having the formula (I) 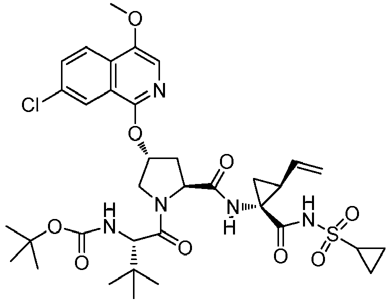 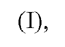 wherein the total dose of the compound of formula (I) administered to the subject is about 100 mg a day.

There is disclosed hereinafter a method of treating an HCV infection comprising the step of administering to a subject in need thereof a formulation comprising a capsule comprising at least one active pharmaceutical ingredient comprising a solubilized compound having the formula (I) 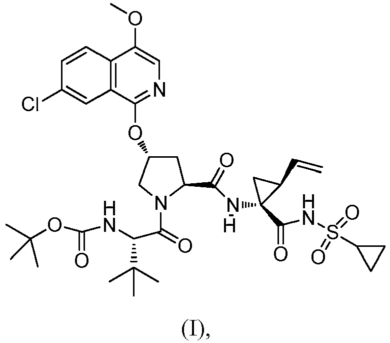 wherein the total dose of the compound of formula (I) administered to the subject is about 50 mg a day.

Other aspects of the present disclosure may include suitable combinations of embodiments disclosed herein.

Yet other aspects and embodiments may be found in the description provided herein.
FIG. 1 illustrates the food effect in dogs of six different formulations of asunaprevir.
FIG. 2 illustrates the pharmacokinetics/bioavailability of capsule and tablet formulations of asunaprevir in dogs.
FIG. 3 illustrates the bioavailability in dogs of soft gel capsule formulations of asunaprevir with varying amounts of polysorbate 80.
FIG. 4 illustrates the relative bioavailability of asunaprevir formulations in fasted and fed humans.

As used in the present specification, the following terms have the meanings indicated:
The term "therapeutically effective amount," as used herein, refers to the total amount of each active component that is sufficient to show a meaningful patient benefit, e.g., a sustained reduction in viral load. When applied to an individual active ingredient, administered alone, the term refers to that ingredient alone. When applied to a combination, the term refers to combined amounts of the active ingredients that result in the therapeutic effect, whether administered in combination, serially, or simultaneously.

The term "treating" refers to: (i) preventing a disease, disorder or condition from occurring in a patient that may be predisposed to the disease, disorder, and/or condition but has not yet been diagnosed as having it; (ii) inhibiting the disease, disorder, or condition, i.e., arresting its development; and (iii) relieving the disease, disorder, or condition, i.e., causing regression of the disease, disorder, and/or condition.

The terms "active pharmaceutical ingredient" and "API," as used herein, refer to asunaprevir.

As used herein, the term "solubilizer" refers to any pharmaceutically acceptable agent that can dissolve API. In the present disclosure the solubilizer dissolves the API and forms the internal phase of an oil-in-water emulsion in which the API is incorporated in the oil droplet following aqueous dilution.

As used herein, the term "surfactant" refers to any pharmaceutically acceptable agent that provides a finer emulsion droplet size following aqueous dilution. As used herein, the term "stabilizer" refers to any pharmaceutically acceptable agent which minimizes oxidative pathways for the degradation of API.

The formulation solutions described herein may be encapsulated as a solution in soft or hard capsules manufactured from various materials including gelatin, hydroxypropyl methylcellulose (HPMC), cellulose, methylcellulose, starch, and other materials. The two-piece capsules may be banded, e.g., with a gelatin-based solution for hard gelatin capsules, or an HPMC-based solution for HPMC capsules. Soft gelatin capsule shells may contain one or more appropriate plasticizers such as glycerin, sorbitol, propylene glycol or others to impart suitable encapsulation, elasticity, mechanical strength, stability and dissolution properties. In addition, the hard or soft gelatin capsule shell may contain and/or be imprinted with various colorants and/or opacifiers.

The formulation solutions described herein may contain one or more of various flavoring agents (e.g., cherry, berry, mint, vanilla, and the like) and/or sweetening agents (e.g., sucrose, sorbitol, mannitol, fructose, dextrose, saccharin, aspartame, acesulfame potassium, and the like) to enhance palatability of the dosage form.

There is disclosed hereinafter a composition comprising a therapeutically effective amount of a compound of formula (III) 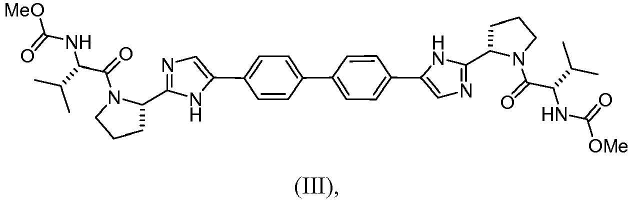 or a pharmaceutically acceptable salt thereof in combination with a formulation comprising a capsule comprising at least one active pharmaceutical ingredient comprising a solubilized compound of formula (I). The ratio of the compound of formula (III), or a pharmaceutically acceptable salt thereof, to the compound of formula (I) may be between about 1:3 and about 3:1; is between about 1:2.5 and about 2.5:1 or about 1:1. The compound of formula (III), or a pharmaceutically acceptable salt thereof, to the compound of formula (I) may be about 1:2.5 or about 2.5:1.

The composition comprising the compound of formula (III), or a pharmaceutically acceptable salt thereof, and the formulation of compound (I) may further comprises one or two additional compounds having anti-HCV activity. At least one of the additional compounds may be an interferon or a ribavirin. The interferon may be selected from interferon alpha 2B, pegylated interferon alpha, consensus interferon, interferon alpha 2A, interferon lambda, pegylated interferon lambda, and lymphoblastoid interferon tau.

The present disclosure provides a composition comprising a therapeutically effective amount of a compound of formula (III), or a pharmaceutically acceptable salt thereof; a formulation comprising a capsule comprising at least one active pharmaceutical ingredient comprising a solubilized compound of formula (I); and a therapeutically effective amount of a compound effective to inhibit the function of HCV polymerase. The compound effective to inhibit the function of HCV polymerase may be selected from PSI-6130, PF-00868554, ANA-598, IDX-375, IDX-184, INX-189, BI-207127, PSI-7851, PSI-938, PSI-879, VCH-759, VCH-916, VCH-222, BMS-929075, GS-9190, ABT-333, and ABT-072. The compound effective to inhibit the function of HCV polymerase may be the compound of formula (IV) 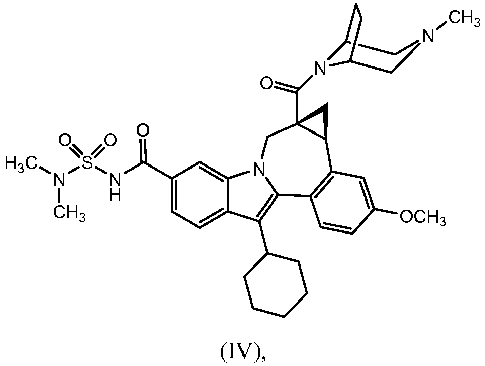 or a pharmaceutically acceptable salt thereof.

The molar ratio of the compound of formula (III), or a pharmaceutically acceptable salt thereof, to the compound of formula (I), to the compound of formula (IV), or a pharmaceutically acceptable salt thereof, may be about 1:20:5 or about 1 :250: 1000.

### EXAMPLES

The present disclosure will now be described in connection with certain embodiments which are not intended to limit its scope. On the contrary, the present disclosure covers all alternatives and modifications as can be included within the scope of the claims. Thus, the following examples, which include specific embodiments, will illustrate one practice of the present disclosure, it being understood that the examples are for the purposes of illustration of certain embodiments and are presented to provide what is believed to be the most useful and readily understood description of its procedures and conceptual aspects. Example 1 is illustrative of the present invention and examples 2-6 do not form part of the invention but represent background art that is useful for understanding the invention.

Asunaprevir can be prepared following the procedure described in U.S. Patent No. 6,995,174, issued February 7, 2006.

The following example is a typical procedure for manufacturing various aspects and embodiments of the present disclosure using mono-, di- and triglycerides of medium-chain fatty acids (e.g., CAPTEX® 355 and CAPMUL® MCM) with the surfactant polyoxyethylene sorbitan monooleate (polysorbate 80) and the antioxidant butylated hydroxytoluene:
1. Transfer the medium-chain triglyceride into the batching vessel.
2. Add the medium-chain mono/diglycerides and surfactant such as polyoxyethylene sorbitan monooleate (polysorbate 80) to the solution in Step 1.
3. Mix the batch from Step 2 to give a homogeneous solution.
4. Add butylated hydroxytoluene to the solution from Step 3 and mix to dissolve.
5. Add API to the solution in Step 4 and mix to dissolve.
6. Encapsulate the solution from Step 6 into a soft or hard gelatin capsules.
7. Dry the soft gelatin capsules to appropriate hardness or apply a band to the hard gelatin capsules.

| *Example 1 (illustrative of the present invention)* | | |
|---|---|---|
| *Ingredient* | *mg*/*capsule* | *Percent (w*/*w)* |
| API | 100 | 20 |
| Medium-Chain Triglycerides | 150 | 30 |
| Medium-Chain Mono/Diglycerides | 150 | 30 |
| Polyoxyethylene Sorbitan Monooleate | 99.5 | 19.9 |
| Butylated Hydroxytoluene | 0.5 | 0.1 |
| | | |
| Total | 500 | 100 |

The following example is a typical procedure for manufacturing various aspects and embodiments of the present disclosure using polyoxyethylated glycerides (linoleoyl polyoxylglycerides, e.g., LABRAFIL® 1944CS) with the surfactant polyoxyethylene sorbitan monooleate (Polysorbate 80) and the antioxidant butylated hydroxytoluene:
1. Transfer the oleoyl polyoxylglycerides into the batching vessel.
2. Add the surfactant such as polyoxyethylene sorbitan monooleate (polysorbate 80) to the solution in Step 1.
3. Mix the batch from Step 2 to give a homogeneous solution.
4. Add butylated hydroxytoluene to the solution from Step 3 and mix to dissolve.
5. Add API to the solution in Step 4 and mix to dissolve.
6. Encapsulate the solution from Step 6 into a soft or hard gelatin capsules.
7. Dry the soft gelatin capsules to appropriate hardness or apply a band to the hard gelatin capsules.

| *Example 2 (not part of the invention)* | | |
|---|---|---|
| *Ingredient* | *mg*/*capsule* | *Percent (w*/*w)* |
| API | 100 | 20 |
| Oleoyl Polyoxylglycerides | 350 | 70 |
| Polyoxyethylene Sorbitan Monooleate | 49.5 | 9.9 |
| Butylated Hydroxytoluene | 0.5 | 0.1 |
| | | |
| Total | 500 | 100 |

An additional example of a formulation of the present disclosure using mono, di and triglycerides of medium-chain fatty acids (e.g., C® 355 and CAPMUL® MCM) with the surfactant Polyoxyl 35 Hydrogenated Castor Oil (e.g., Cremophor® EL) and the antioxidant butylated hydroxytoluene is shown in Example 3:

| *Example 3 (not part of the invention)* | | |
|---|---|---|
| *Ingredient* | *mg*/*capsule* | *Percent (w*/*w)* |
| API | 100 | 20 |
| Medium-Chain Triglycerides | 150 | 30 |
| Medium-Chain Mono/Diglycerides | 150 | 30 |
| Polyoxyl 35 Hydrogenated Castor Oil | 99.5 | 19.9 |
| Butylated Hydroxytoluene | 0.5 | 0.1 |
| | | |
| Total | 500 | 100 |

An additional example of a formulation of the present disclosure using triglycerides of medium-chain fatty acids (e.g., CAPTEX® 355) and a propylene glycol ester of a medium-chain fatty acid such as propylene glycol monocaprylate (e.g., CAPMUL® PG-8 or Capryol™ 90) with the surfactant polyoxyethylene sorbitan monooleate (Polysorbate 80) and the antioxidant butylated hydroxytoluene is shown in Example 4:

| *Example 4 (not part of the invention)* | | |
|---|---|---|
| *Ingredient* | *mg*/*capsule* | *Percent (w*/*w)* |
| API | 100 | 20 |
| Medium-Chain Triglycerides | 200 | 40 |
| Propylene Glycol Monocaprylate | 100 | 20 |
| Polyoxyethylene Sorbitan Monooleate | 99.5 | 19.9 |
| Butylated Hydroxytoluene | 0.5 | 0.1 |
| | | |
| Total | 500 | 100 |

An additional example of a formulation of the present disclosure using triglycerides of medium-chain fatty acids (e.g., CAPTEX® 355) and a propylene glycol ester of a medium-chain fatty acid such as propylene glycol monolaurate (e.g., CAPMUL® PG-12 or Lauroglycol™ 90) with the surfactant polyoxyethylene sorbitan monooleate (Polysorbate 80) and the antioxidant butylated hydroxytoluene is shown in Example 5:

| *Example 5 (not part of the invention)* | | |
|---|---|---|
| *Ingredient* | *mg*/*capsule* | *Percent (w*/*w)* |
| API | 100 | 20 |
| Medium-Chain Triglycerides | 200 | 40 |
| Propylene Glycol Monolaurate | 100 | 20 |
| Polyoxyethylene Sorbitan Monooleate | 99.5 | 19.9 |
| Butylated Hydroxytoluene | 0.5 | 0.1 |
| | | |
| Total | 500 | 100 |

An additional example of a formulation of the present disclosure using polyoxyethylated glycerides (linoleoyl polyoxylglycerides, e.g., LABRAFIL® 2125CS) with the surfactant polyoxyethylene sorbitan monooleate (Polysorbate 80) and the antioxidant butylated hydroxytoluene is shown in Example 6:

| *Example 6 (not part of the invention)* | | |
|---|---|---|
| *Ingredient* | *mg*/*capsule* | *Percent (w*/*w)* |
| API | 100 | 20 |
| Linoleoyl Polyoxylglycerides | 350 | 70 |
| Polyoxyethylene Sorbitan Monooleate | 49.5 | 9.9 |
| Butylated Hydroxytoluene | 0.5 | 0.1 |
| | | |
| Total | 500 | 100 |

### Example 7: In Vivo Studies

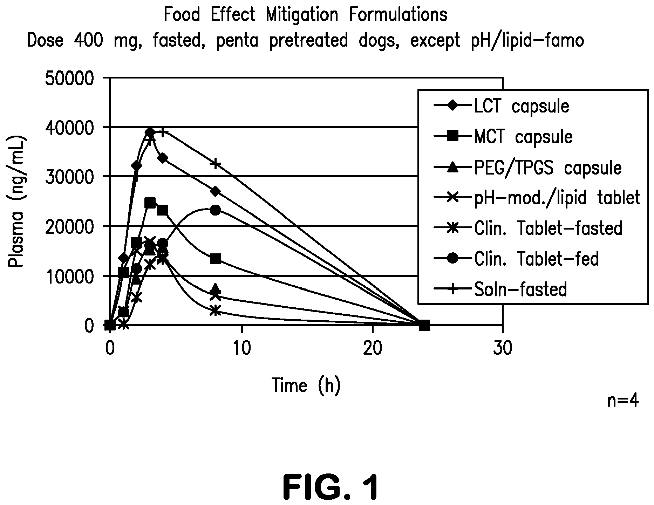

Table 1 and Figure 1 present the results from a PK study conducted in dogs to compare the PK of four formulations developed in effort to increase the oral bioavailability of asunaprevir and mitigate the food effect observed for the dry granulation tablet formulation in humans. The capsule formulations represent three different solubilized approaches in which the drug was fully solubilized as a solution in either (1) a long-chain fatty acid triglyceride-based lipid self-emulsifying system containing 20% w/w drug load with LABRAFIL® M1944CS and polysorbate 80, designated as "LCT Capsule", (2) a medium-chain triglyceride-based lipid self-emulsifying system containing 20% w/w drug load with CAPTEX® 355, CAPMUL® MCM and polysorbate 80, designated as "MCT Capsule", and (3) a non-lipid system containing 20% w/w drug load with polyethylene glycol 3350 and vitamin E TPGS (i.e., d-α-tocopheryl polyethylene glycol 1000 succinate). The formulations (1) to (3) do not form part of the invention but represent background art that is useful for understanding the invention.

Each capsule formulation was administered at a 400-mg dose to fasted dogs pre-treated with pentagastrin. In addition, a tablet formulation was evaluated containing 50% w/w drug load with Gelucire® 53/10 and the pH-modifier sodium bicarbonate. The tablet was administered at a 400-mg dose to fasted dogs pre-treated with famotidine to increase the pH of the GI tract. The PK of these 4 formulations was compared to the dry granulation tablet administered at a 400-mg dose to fasted dogs pre-treated with pentagastrin in one study arm, and at a 400-mg dose to fed dogs pre-treated with pentagastrin in a second study arm. Each of 4 dogs received all 6 formulation treatments (one week washout period before dosing each subsequent formulation).

As shown in Table 1 and Figure 1, the long-chain fatty acid triglyceride-based lipid (LCT Capsule) and medium-chain triglyceride-based lipid (MCT Capsule) self-emulsifying systems provided superior oral absorption of asunaprevir in the dogs, increasing the oral bioavailability in the fasted-state by 3- to 5-fold. These LCT and MCT lipid-based solubilized capsule formulations also provided the most pronounced reduction in the significant food effect observed for the clinical dry granulation tablet formulation as represented by the ratio of the AUC for the formulation dosed in the fasted-state compared to the AUC for the tablet dosed in the fed-state.

**TABLE 1**

| *Dog PK Results from Formulation Screening Study* | | | | | | | | | |
|---|---|---|---|---|---|---|---|---|---|
| Formulation | Pre-Treatment | Cmax (ng/mL) | | Tmax (h) | AUC-24h (ng·h/mL) | | BA (%) | | Fed:Fasted Ratio |
| | | Mean | SD | Median | Mean | SD | Mean | SD | |
| HGC - 70% LABRAFIL M1944CS : 10% Polysorbate 80 (LCT Capsule) | Pentagastri n & Fasted | 43126 | 13761 | 2.5 | 440671 | 391699 | 528 | 470 | 0.69 |
| HGC - 20% Polysorbate 80 : 30% CAPTEX 355:30% CAPMUL MCM (MCT Capsule) | Pentagastri n & Fasted | 29033 | 21362 | 2.5 | 243511 | 219862 | 292 | 264 | 1.25 |
| HGC - 40% PEG 3350 : 40% TPGS (PEG/TPGS Capsule) | Pentagastri n & Fasted | 21180 | 6122 | 3 | 143844 | 77103 | 173 | 92 | 2.11 |
| Dry Granulation Tablet: Gelucire 53/10:Sodiu m Bicarbonate (pH-Mod./Lipid Tablet) | Famotidin e & Fasted | 19542 | 8756 | 2.5 | 131503 | 94509 | 158 | 113 | 2.31 |
| Dry Granulation Tablet (Clin. Tablet-Fasted) | Pentagastri n & Fasted | 14420 | 8305 | 4 | 83385 | 59101 | | | 3.64 |
| Dry Granulation Tablet (Clin. Tablet - Fed) | Pentagastri n & Fed | 29924 | 5570 | 6 | 303526 | 97245 | 364 | 117 | |

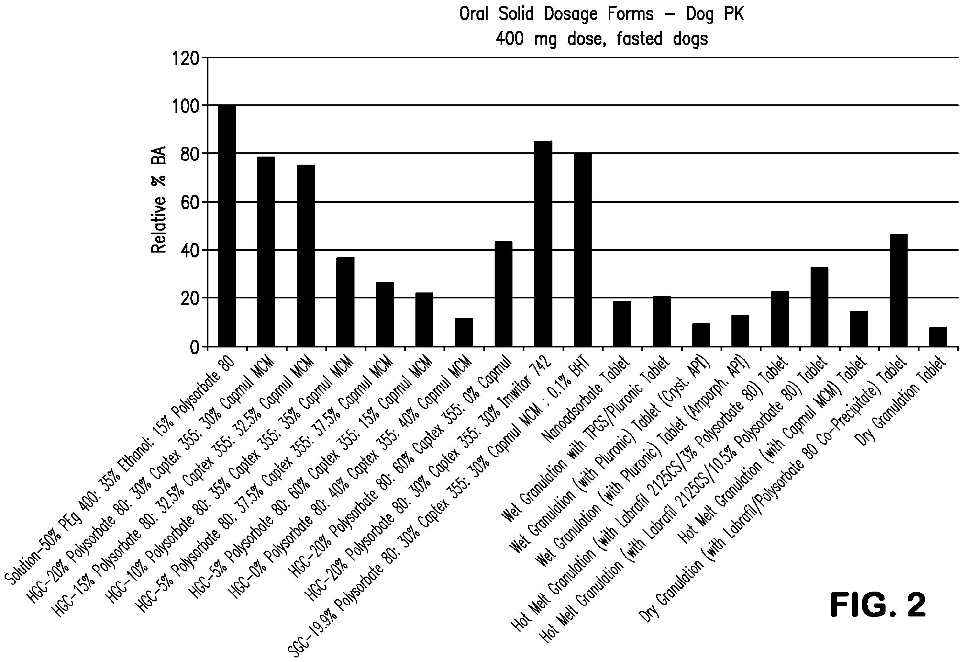

Table 2 and Figure 2 present the cumulative results from several studies conducted in dogs to assess the PK of numerous capsule and tablet formulations of asunaprevir developed in an effort to increase the oral bioavailability and mitigate the food effect observed for the dry granulation tablet formulation in humans. All PK results shown were generated in pentagastrin-treated and fasted dogs dosed 400-mg of asunaprevir from the various formulations.

As noted, eight tablet formulations utilizing various approaches were developed and evaluated in an effort to increase the bioavailability of asunaprevir and mitigate the significant food effect observed for the dry granulation tablet. These tablet formulations do not form part of the invention but represent background art that is useful for understanding the invention. Compared to the dry granulation tablet which demonstrates a bioavailability of 8% relative to the solution formulation, some improvement in PK was observed for the various alternative tablet approaches, with relative bioavailabilities ranging from 9.1 to 46.5%.

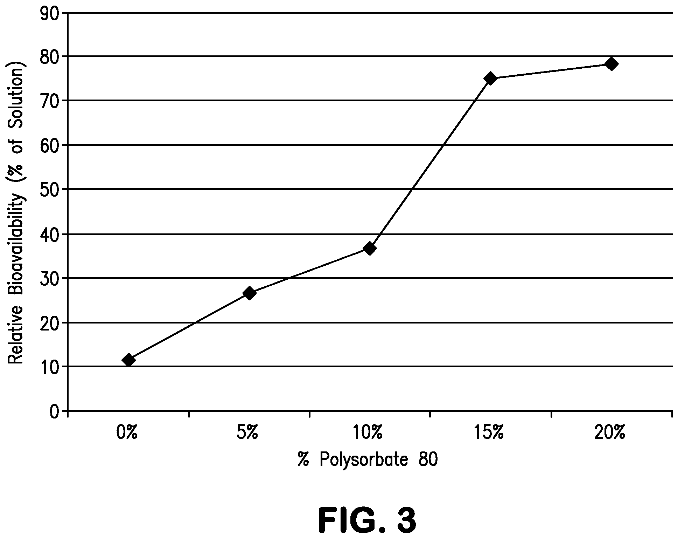

Several variations of the medium-chain triglyceride-based lipid self-emulsifying system were evaluated to optimize bioavailability from the solubilized capsule approach. The hard gelatin capsule (HGC) formulations do not form part of the invention but represent background art that is useful for understanding the invention while the soft gelatin capsule formulation is illustrative of the present invention. As shown in Table 2 and Figure 2, a formulation composition containing 20% w/w drug load with 30% w/w medium-chain triglyceride (e.g., CAPTEX® 355), 30% w/w medium-chain mono/diglycerides (e.g., CAPMUL® MCM or Imwitor® 742) and 15-20% w/w polysorbate 80 provided the highest bioavailability of asunaprevir (relative to solution formulation) at 75-85%. As also noted in Table 2 and Figure 3, this formulation encapsulated in either a hard gelatin capsule or soft gelatin capsule provided comparably superior PK results. Unexpectedly, although there was no significant difference in drug solubility, ease of self-emulsification or emulsion droplet size distribution following aqueous dilution observed with the medium-chain triglyceride based system containing lower than 15% w/w polysorbate 80, the systems with 15% or 20% polysorbate 80 provided the highest relative bioavailabilities as shown in Table 2 and Figures 2 and 3.

**TABLE 2**

| *Cumulative Dog PK Results from Formulation Study* | | | | | | | | |
|---|---|---|---|---|---|---|---|---|
| Formulation | Pre-Treatment | Cmax (ng/mL) | | Tmax (h) | AUC-24h (ng·h/mL) | | BA (%) | |
| | | Mean | SD | Median | Mean | SD | Mean | SD |
| Solution - 50% PEG 400 : 35% Ethanol : 15% Polysorbate 80 | Pentagstrin & Fasted | 43126 | 13761 | 3 | 504019 | 265726 | 100 | 52.7 |
| HGC - 20% Polysorbate 80 : 30% CAPTEX 355 : 30% CAPMUL MCM | Pentagstrin & Fasted | 40183 | 10619 | 4 | 395137 | 173805 | 78.4 | 34.5 |
| HGC - 15% Polysorbate 80 : 32.5% CAPTEX 355 : 32.5% CAPMUL MCM | Pentagstrin & Fasted | 40333 | 8396 | 3 | 378133 | 136931 | 75.0 | 27.2 |
| HGC - 10% Polysorbate 80 : 35% CAPTEX 355 : 35% CAPMUL MCM | Pentagstrin & Fasted | 24125 | 8424 | 3 | 185419 | 112587 | 36.8 | 22.3 |
| HGC - 5% Polysorbate 80 : 37.5% CAPTEX 355 : 37.5% CAPMUL MCM | Pentagstrin & Fasted | 20100 | 11392 | 3 | 134178 | 83467 | 26.6 | 16.6 |
| HGC - 5% Polysorbate 80 : 60% CAPTEX 355 : 15% CAPMUL MCM) | Pentagstrin & Fasted | 15366 | 7695 | 2 | 109207 | 86876 | 21.7 | 17.2 |
| HGC - 0% Polysorbate 80 : 40% CAPTEX 355 : 40% CAPMUL MCM) | Pentagstrin & Fasted | 10066 | 4026 | 3 | 58241 | 25819 | 11.6 | 5.1 |
| HGC - 20% Polysorbate 80 : 60% CAPTEX 355 : 0% CAPMUL | Pentagstrin & Fasted | 26400 | 4359 | 4 | 217742 | 33535 | 43.2 | 6.7 |
| HGC - 20% Polysorbate 80 : 30% CAPTEX 355 : 30% Imwitor 742 | Pentagstrin & Fasted | 41025 | 4613 | 3 | 429537 | 102361 | 85.2 | 20.3 |
| SGC - 19.9% Polysorbate 80 : 30% CAPTEX :355 30% CAPMUL MCM : 0.1% BHT | Pentagstrin & Fasted | 42675 | 15966 | 3 | 402129 | 183224 | 79.8 | 36.4 |
| Nanoadsorbate Tablet - PVP:K12:TPGS: Cabosil | Pentagstrin & Fasted | 14367 | 4477 | 2 | 92814 | 51633 | 18.4 | 10.2 |
| Wet Granulation with TPGS/Pluronic Tablet | Pentagstrin & Fasted | 14382 | 3776 | 3 | 104507 | 28685 | 20.7 | 5.7 |
| Wet Granulation (with Pluronic) Tablet (Cryst. API) | Pentagstrin & Fasted | 8945 | 6744 | 3 | 45712 | 38370 | 9.1 | 7.6 |
| Wet Granulation (with Pluronic) Tablet (Amorph. API) | Pentagstrin & Fasted | 10402 | 4941 | 2.5 | 63446 | 31814 | 12.6 | 6.3 |
| Hot Melt Granulation (with LABRAFIL 2125CS/3% Polysorbate 80) Tablet | Pentagstrin & Fasted | 17376 | 3218 | 3 | 112885 | 34533 | 22.4 | 6.9 |
| Hot Melt Granulation (with LABRAFIL 2125CS/10.5% Polysorbate 80) Tablet | Pentagstrin & Fasted | 20298 | 3551 | 2.5 | 162902 | 62210 | 32.3 | 12.3 |
| Hot Melt Granulation (with CAPMUL MCM) Tablet | Pentagstrin & Fasted | 10655 | 2501 | 3 | 72608 | 20277 | 14.4 | 4.0 |
| Dry Granulation (LABRAFIL/Poly sorbate 80 Co-Precipitate) Tablet | Pentagstrin & Fasted | 28844 | 8503 | 3 | 234373 | 87155 | 46.5 | 17.3 |
| Dry Granulation Tablet | Pentagstrin & Fasted | 8694 | 586 | 2.5 | 40519 | 8796 | 8.0 | 1.8 |

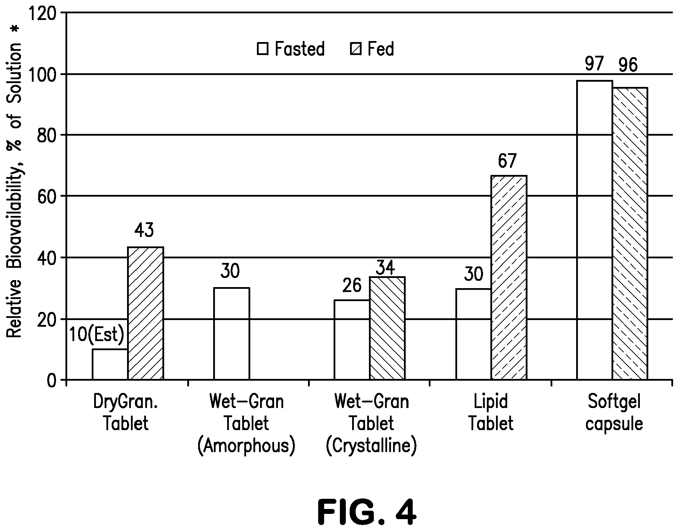

Figure 4 and Tables 3 and 4 present the results from a relative bioavailability clinical study conducted in humans to evaluate the PK and effect of fasted versus fed conditions on the oral absorption of various formulations of asunaprevir. The formulations evaluated included (1) film-coated 200-mg strength tablet manufactured from a dry granulation containing 50% w/w drug load with microcrystalline cellulose, croscarmellose sodium, sodium lauryl sulfate and magnesium stearate and film-coated with Opadry®-II (a white non-functional coating system), (2) film-coated 200-mg strength tablet manufactured from a wet granulation containing 50% w/w drug load (amorphous API) with microcrystalline cellulose, lactose monohydrate, croscarmellose sodium, polaxamer 188, vitamin E polyethylene glycol succinate and magnesium stearate) and film-coated with Opadry®-II, (3) film-coated 200-mg strength tablet manufactured from a wet granulation containing 50% w/w drug load (crystalline API) with microcrystalline cellulose, lactose monohydrate, croscarmellose sodium, polaxamer 188, vitamin E polyethylene glycol succinate and magnesium stearate) and film-coated with Opadry®-II, (4) uncoated 100-mg strength tablet manufactured from a dry granulation containing microcrystalline cellulose, croscarmellose sodium, linoleoyl polyoxyglycerides, polysorbate 80, magnesium aluminometasilicate, anhydrous dibasic calcium phosphate, magnesium stearate, and butylated hydroxytoluene (BHT), and (5) 100-mg strength soft gelatin capsule containing 20% w/w drug load dissolved in a mixture of medium-chain triglycerides, glycerol monocaprylocaprate, polysorbate 80, and butylated hydroxytoluene (BHT). These formulations are not part of the invention and represent background art that is useful for understanding the invention.

The study design included randomization of eight formulation/dietary-state (fed vs. fasted) treatments (A, B, C, D, E, F, G and H), with each of 35 subjects randomized to receive five single doses consisting of the 200-mg asunaprevir dry granulation tablet fed-state "A" (Reference) and four additional formulation/dietary-state treatments either fed (standard meal) or fasted, randomized from B-H. Single-dose pharmacokinetic (PK) parameters were derived from the plasma concentration versus time profiles. Relative bioavailabilities as shown in Figure 4 and Tables 3 and 4 are calculated from the AUC(INF) values for each treatment compared to the AUC(INF) value of a solution formulation of asunaprevir (20 mg/mL asunaprevir in 50% w/w PEG 400 / 35% w/w Ethanol/ 15% w/w Polysorbate 80) dosed at 200-mg in a separate clinical study. The relative bioavailability of the reference dry granulation tablet dosed at 200-mg in the fasted-state was estimated from PK data also generated in a separate clinical study.

As shown in Figure 3 and Table 3, the bioavailability of asunaprevir from the soft gelatin capsule in the fasted-state was approximately 3 to 10-fold higher compared to the tablet formulations. In addition, as shown in Figure 3 and Table 4, the bioavailability from the soft gelatin capsule in the fed-state was approximately 1.4 to 2.8-fold higher compared to the tablet formulations. Furthermore, the results indicated substantially complete mitigation of food effect for the soft gelatin capsule formulation in contrast to the significant effect of food on oral bioavailability demonstrated by the tablet formulations. Substantially complete mitigation of the food effect is defined as a formulation that has a fed fasted ratio of 1.5 or less; or 1.25 or lower or 1.0 or lower.

**TABLE 3**

| *Results from Human Bioavailability Study* | | | | | |
|---|---|---|---|---|---|
| PK Parameter | Treatment B: Wet Granulation Tablet (Amorphous API) | Treatment C: Wet Granulation Tablet (Crystalline API) | Treatment E: Lipid Tablet | Treatment G: Soft Gelatin Capsule | Solution* |
| Cmax-GM (CV%) | 45.7 (160) | 40.9 (128) | 42 (87) | 252 (106) | 367 (72) |
| AUCinf-GM (CV%) | 328 (123) | 285 (88) | 324 (81) | 1060 (70) | 1087 (63) |
| AUCinf Relative to Solution | 30% | 26% | 30% | 97% | |

| | | | | | |
|---|---|---|---|---|---|
| Treatment B: 1 x 200 mg Asunaprevir Wet Granulation Tablet with Amorphous API (Fasted) Treatment C: 1 x 200 mg Asunaprevir Wet Granulation Tablet with Crystalline API (Fasted) Treatment E: 1 x 200 mg Asunaprevir Lipid Tablet (Fasted) Treatment G: 1 x 200 mg Asunaprevir Soft Gelatin Capsule (Fasted) * Solution: (Formulation : 20 mg/mL asunaprevir in 50% w/w PEG 400 /35% w/w Ethanol/ 15% w/w Polysorbate 80 | | | | | |

**TABLE 4**

| *Results from Human Bioavailability Study* | | | | | |
|---|---|---|---|---|---|
| PK Parameter | Treatment A: Dry Granulation Tablet | Treatment D: Wet Granulation Tablet (Crystalline API) | Treatment F: Lipid Tablet | Treatment H: Soft Gelatin Capsule | Solution* |
| Cmax-GM (CV%) | 61.8 (97) | 51.9(101) | 114 (77) | 288(59) | 367 (72) |
| AUCinf-GM (CV%) | 470 (61) | 366 (66) | 725 (53) | 1040 (41) | 1087 (63) |
| AUCinf Relative to Solution | 43% | 34% | 67% | 96% | |

| | | | | | |
|---|---|---|---|---|---|
| Treatment A: 1 x 200 mg Asunaprevir Dry Granulation Tablet (Fed) Treatment D: 1 x 200 mg Asunaprevir Wet Granulation Tablet with Crystalline API (Fed) Treatment F: 1 x 200 mg Asunaprevir Lipid Tablet (Fed) Treatment H: 1 x 200 mg Asunaprevir Soft Gelatin Capsule (Fed) * Solution: PK data from separate clinical study (Formulation : 20 mg/mL asunaprevir in 50% w/w PEG 400 / 35% w/w Ethanol / 15% w/w Polysorbate 80 | | | | | |

Table 5 shows the chemical stability of asunaprevir in the formulated capsule with varying amounts of BHT. The formulations comprising BHT are illustrative of the present invention and the last formulation in Table 5 is a reference formulation that does not form part of the invention. As shown below, using as little as 0.02% BHT provides suitable chemical stability with less than 2% degradation, including at least 6-months at accelerated stability conditions such as 25°C/60% RH (open dish) and 40°C/75% RH in various HDPE bottles and blister packages.

**TABLE 5**

| *Effect of Butylated Hydroxytoluene (BHT) Level on the Stability of a Formulation Solution* | | | | | | | |
|---|---|---|---|---|---|---|---|
| Formulation | % BHT | 6 Months 50°C (Solution-in- Vial) | | 6 Months 40°C/75% RH (Capsule) | | | |
| | | API Potency Retention | % Deg. #1 | API Potency Retention | % Deg. #1 | % Deg. #2 | % Deg. #3 |
| 20% API 30% Medium-Chain Triglycerides 30% Medium-Chain Mono/Diglycerides 19.9% Polysorbate 80 | 0.1 | 103.1%* | < 0.05* | 99.5%* | < 0.05* | 0.15* | 0.18* |
| 20% API 30% Medium-Chain Triglycerides 30% Medium-Chain Mono/Diglycerides 19.95% Polysorbate 80 | 0.05 | 100.0% | < 0.05 | 99.1% | < 0.05 | 0.15 | 0.18 |
| 20% API 30% Medium-Chain Triglycerides 30% Medium-Chain Mono/Diglycerides 19.98% Polysorbate 80 | 0.02 | 98.7% | < 0.05 | 98.4% | < 0.05 | 0.15 | 0.19 |
| 20% API 30% Medium-Chain Triglycerides 30% Medium-Chain Mono/Diglycerides 19.99% Polysorbate 80 | 0.01 | 73.9% | 14.2 | 98.7% | < 0.05 | 0.14 | 0.19 |
| 20% API 30% Medium-Chain Triglycerides 30% Medium-Chain Mono/Diglycerides 20% Polysorbate 80 | 0 | 3.8% | 68.4 | 86.0% | 15.1 | 0.75 | 0.38 |

| | | | | | | | |
|---|---|---|---|---|---|---|---|
| *Average of 7 formulations tested | | | | | | | |

### Dosing Schedule Studies

A randomized 8-treatment, 5-period incomplete crossover study balanced for first-order carryover effects, was conducted in healthy men and women ages 18-49 years and BMI 18-32 kg/m². Subjects (n=35) each received 200mg doses of the reference dry-granulated tablet formulation with standard meal and four of the following test treatments: formulations 1, 2, 3, and softgel capsule administered fasted and formulations 2, 3, and softgel capsule administered with standard meal. Subjects had periodic safety assessments and were monitored for adverse events (AEs). Blood samples for pharmacokinetics (PK) were collected for 72h post-dose. Noncompartmental PK were derived. Geometric mean ratios (GMR) and 90% confidence intervals (CI) were calculated using general linear mixed-effect models. Post-hoc GMRs and 90% CIs were estimated for 100mg of softgel capsule using normalized exposures from 200mg data. Distributions of softgel capsule steady state AUC (AUCss) at 100mg BID were simulated and evaluated in context of prior exposure-response and exposure-safety assessments.

All AEs were mild/moderate and no discontinuations were due to AEs. Formulations 1, 2, and 3 had lower bioavailability than the reference, unacceptable food effects or both. GMR [90%CI] for fasted softgel capsule was 2.23 [1.86-2.67] for AUC(0-∞) and 4.09 [3.08-5.43] for Cmax. For softgel capsule with food, GMR [90% CI] was 2.60 [2.17-3.19] for AUC(0-∞), indicating minimal food effect (only +17%), and 5.36 [4.03-7.12] for Cmax. Post-hoc comparisons for normalized exposures of softgel capsule at 100mg fasted relative to the 200mg reference tablet, provided GMR [90%CI] of 1.11 [0.929-1.34] for AUC(0-∞) and 2.04 [1.54-2.72] for Cmax. For 100mg softgel capsule fed, GMR [90%CI] was 1.30 [1.09-1.56] for AUC(0-∞) and 2.68 [2.02-3.56] for Cmax. Increased ASV Cmax values with softgel capsule were transient when fed or fasted. Simulations suggested considerable overlap of AUCss between projected softgel capsule exposures at 100mg BID and fed tablet at 200mg BID. Exposure-safety analysis indicated that the probability of ASV-associated ALT elevations would be expected to be similar with 100mg BID softgel relative to 200mg of tablet formulation. Higher but transient ASV Cmax values are unlikely to be clinically relevant as both efficacy and safety events for ASV appear to correlate best with AUCss.

It will be evident to one skilled in the art that the present disclosure is not limited to the foregoing illustrative examples, and that it can be embodied in other specific forms without departing from the essential attributes thereof. It is therefore desired that the examples be considered in all respects as illustrative and not restrictive, reference being made to the appended claims, rather than to the foregoing examples, and all changes which come within the meaning of the claims are therefore intended to be embraced therein.

## Claims

1. A formulation comprising:
(a) at least a compound of formula (I) 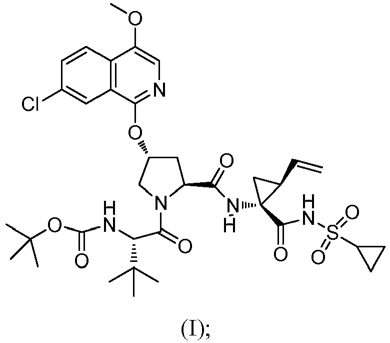
(b) at least one solubilizer which is medium-chain fatty acid triglycerides and a combination of medium-chain fatty acid mono- and diglycerides, wherein the at least one solubilizer is included in the range from 10 to 80% w/w;
(c) at least one surfactant which is polyoxyethylene sorbitan monooleate, wherein the at least one surfactant is included in the range from 15 to 40% w/w; and
(d) at least one stabilizer which is butylated hydroxytoluene.

2. The formulation of claim 1 wherein the at least one active pharmaceutical ingredient is included in the range from 1 to 40% w/w.

3. The formulation of claim 1 wherein the at least one stabilizer is included in the range from 0.05 to 0.2% w/w.

4. The formulation of any one of claims 1 to 3 for use in the treatment of an hepatitis C virus infection.

5. The formulation for use according to claim 4 wherein the formulation comprises a capsule and the total dose of the compound of formula (I) administered to the subject is 200 mg a day.

6. The formulation for use according to claim 5 wherein the compound of formula (I) is administered to the subject in doses of 100 mg two times a day.

7. The formulation for use according to claim 4 wherein the formulation comprises a capsule and the total dose of the compound of formula (I) administered to the subject is 150 mg a day, 100mg a day or 50 mg a day.

## Patentansprüche

1. Formulierung, umfassend:
(a) mindestens eine Verbindung der Formel (I) 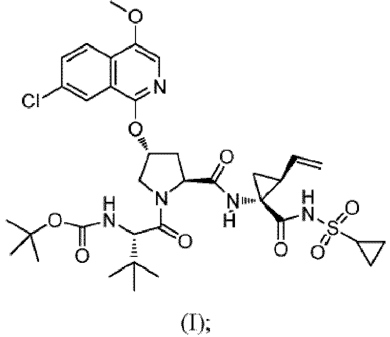
(b) mindestens einen Lösungsvermittler, der mittelkettige Fettsäure-Triglyceride und eine Kombination von mittelkettigen Fettsäure-Mono- und -Diglyceriden ist, wobei der mindestens eine Lösungsvermittler im Bereich von 10 bis 80 Gew.-% enthalten ist;
(c) mindestens ein Tensid, das Polyoxyethylensorbitanmonooleat ist, wobei das mindestens eine Tensid im Bereich von 15 bis 40 Gew.-% enthalten ist; und
(d) mindestens einen Stabilisator, der Butylhydroxytoluol ist.

2. Formulierung nach Anspruch 1, wobei der mindestens eine aktive pharmazeutische Inhaltsstoff im Bereich von 1 bis 40 Gew.-% enthalten ist.

3. Formulierung nach Anspruch 1, wobei der mindestens eine Stabilisator im Bereich von 0,05 bis 0,2 Gew.-% enthalten ist.

4. Formulierung nach einem der Ansprüche 1 bis 3 zur Verwendung bei der Behandlung einer Hepatitis-C-Virusinfektion.

5. Formulierung zur Verwendung nach Anspruch 4, wobei die Formulierung eine Kapsel umfasst und die gesamte Dosis der Verbindung der Formel (I), die dem Probanden verabreicht wird, 200 mg am Tag ist.

6. Formulierung zur Verwendung nach Anspruch 5, wobei die Verbindung der Formel (I) dem Probanden in Dosen von 100 mg zweimal am Tag verabreicht wird.

7. Formulierung zur Verwendung nach Anspruch 4, wobei die Formulierung eine Kapsel umfasst und die gesamte Dosis der Verbindung der Formel (I), die dem Probanden verabreicht wird, 150 mg am Tag, 100 mg am Tag oder 50 mg am Tag ist.

## Revendications

1. Formulation comprenant:
(a) au moins un composé de la formule (I): 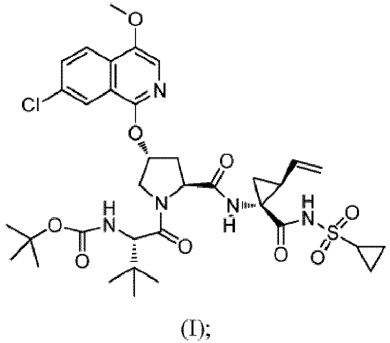
(b) au moins un agent solubilisant qui est des triglycérides d'acides gras à chaîne moyenne et une combinaison de mono- et diglycérides d'acides gras à chaîne moyenne, dans laquelle le au moins un agent solubilisant est inclus dans l'intervalle de 10 à 80% p/p;
(c) au moins un tensioactif qui est un monooléate de polyoxyéthylène sorbitane, dans laquelle le au moins un tensioactif est inclus dans l'intervalle de 15 à 40% p/p; et
(d) au moins un agent stabilisant qui est un hydroxytoluène butylé.

2. Formulation selon la revendication 1, dans laquelle le au moins un ingrédient pharmaceutique actif est inclus dans l'intervalle de 1 à 40% p/p.

3. Formulation selon la revendication 1, dans laquelle le au moins un agent stabilisant est inclus dans l'intervalle de 0,05 à 0,2% p/p.

4. Formulation selon l'une quelconque des revendications 1 à 3 pour une utilisation dans le traitement d'une infection par le virus de l'hépatite C.

5. Formulation pour une utilisation selon la revendication 4, où la formulation comprend une capsule et la dose totale du composé de la formule (I) administrée au sujet est de 200 mg par jour.

6. Formulation pour une utilisation selon la revendication 5, dans laquelle le composé de la formule (I) est administré au sujet dans des doses de 100 mg deux fois par jour.

7. Formulation pour une utilisation selon la revendication 4, où la formulation comprend une capsule et la dose totale du composé de la formule (I) administrée au sujet est de 150 mg par jour, 100 mg par jour ou 50 mg par jour.
